# EUROPEAN PATENT SPECIFICATION

(11) **EP 3 288 466 B1**
(45) Date of publication and mention of the grant of the patent: **12.06.2019**
(21) Application number: 16723863.3
(22) Date of filing: 26.04.2016
(51) Int. Cl.: A61B 10/00, A61B 10/02, G01N 1/00, A01N 1/02, B65D 25/00

(54) **BIOPSY TISSUE HANDLING APPARATUS**
BIOPSIEGEWEBEHANDHABUNGSVORRICHTUNG
APPAREIL DE MANIPULATION DE TISSU DE BIOPSIE

(30) Priority: 27.04.2015 US 201562153284 P
(43) Date of publication of application: 07.03.2018
(73) Proprietor: Cook Medical Technologies LLC, Bloomington, IN 47404 (US)
(72) Inventor: CHANG, Kenneth, Cerritos, CA 90703 (US)
(74) Representative: Garratt, Peter Douglas
(86) International application number: PCT/US2016/029337
(87) International publication number: WO 2016/176195

(56) References cited:
- WO-A1-2013/192606
- WO-A2-2007/021904
- WO-A2-2011/094577
- US-A- 5 817 032
- US-A1- 2011 003 324
- US-A1- 2013 324 882

## Description

### BACKGROUND OF THE INVENTION

### 1. Field of the Invention

The present description relates to a biopsy tissue handling apparatus, biopsy kits, and methods of processing tissue specimens.

### 2. Description of the Related Art

Endoscopic ultrasound biopsy needles are utilized for fine needle biopsy (FNB) of liver tissue, submucosal lesions, mediastinal masses, lymph nodes and intraperitoneal masses within or adjacent to the gastrointestinal tract. Once the tissue sample has been obtained within the needle, the physician needs to transfer the specimen to the pathology lab in an effective and efficient manner. There are currently methods in which to make this transition of the specimen from the needle to the pathologist. However, this is often a tedious task as care must be taken to not fracture the frail specimens (fresh liver specimens are especially frail). Often the transition to pathology fragments the sample making evaluation much more difficult. This failure to securely transition the core sample to the pathologist can provide the impression that the needle is suboptimal and itself is fragmenting the tissue. Additionally, separating blood clots from the specimen(s) to be evaluated is not trivial.

In view of the difficulty in transferring intact and clot-free tissue specimens from a biopsy needle to a pathology lab using current techniques, there is a need for improved devices and techniques for handling, processing, and transferring fresh tissue specimens without damaging or degrading the specimens.

### SUMMARY OF THE INVENTION

The invention generally relates to a kit and device for processing a tissue specimen (e.g., liver tissue) obtained from a biopsy needle to separate a tissue core-destined for a pathology lab-from unwanted clot material without damage to the tissue core. In one aspect, this description provides a biopsy kit including a biopsy tissue handling device having a first well, a second well, a third well, and a channel network connecting the wells such that there is fluid communication between the wells. The first, second, and third wells each has a bottom, a side wall, and an upward facing opening. In certain embodiments the channel network has first, second, and third channels, each channel having a first end connected, respectively, to the first, second, or third wells, where the second ends of the channels connect to form an intersection point. In other embodiments, the channel network has first and second channels, each channel having first ends connected, respectively, to the first and second wells, where the second ends of the first and second channels connect to the third well such that the third well comprises a part of the fluid communication between the first and second wells.

In another aspect, the present description provides a method of processing a tissue specimen by obtaining a tissue specimen having a core and a clot, transferring the tissue specimen to a biopsy tissue handling device according to the invention, manipulating the tissue specimen to separate the tissue core from the clot. In certain embodiments, the separated tissue core is transferred to a pathology cassette.

US patent 2011/0003324 describes a kit according to the preamble of claim 1.

### BRIEF DESCRIPTION OF THE DRAWINGS

FIG. 1 illustrates one embodiment of a biopsy tissue handling device.
FIG. 2 illustrates another embodiment of a biopsy tissue handling device.
FIG. 3 illustrates a tissue specimen in a channel of a biopsy tissue handling device with a cutting tool positioned above the specimen.
FIG. 4A illustrates an embodiment of the first well having a grate in the first well.
FIG. 4B illustrates an embodiment of the second well having a sponge in the second well.
FIG. 5A illustrates a tissue specimen in transition between the first well into a channel of the biopsy tissue handling device.
FIG. 5B illustrates one embodiment of a tissue maneuvering tool with a tissue specimen.
FIG. 5C illustrates a tissue core positioned on a sponge after having been transferred to the second well.
FIG. 5D illustrates a tissue core positioned between two sponges after having been transferred from the biopsy tissue handling device to a pathology cassette.
FIG. 6 illustrates another embodiment of a biopsy tissue handling device.
FIG. 7A illustrates an example of a biopsy needle.
FIG. 7B illustrates an example of a syringe for use with a biopsy needle.
FIGS. 8A and 8B illustrate an example of a tray for packaging a biopsy needle and syringe. FIG. 8A also shows the outline of how an exemplary biopsy needle and syringe may fit into a packaging tray.
FIG. 9 illustrates an embodiment of a biopsy tissue handling device configured as a packaging tray for a biopsy needle and syringe.

### DETAILED DESCRIPTION

The embodiments are described with reference to the drawings in which like elements are referred to by like numerals. The relationship and functioning of the various elements of the embodiments are better understood by the following detailed description. It should also be understood that the drawings are not to scale and in certain instances details have been omitted, which are not necessary for an understanding of the embodiments, such as conventional details of fabrication and assembly.

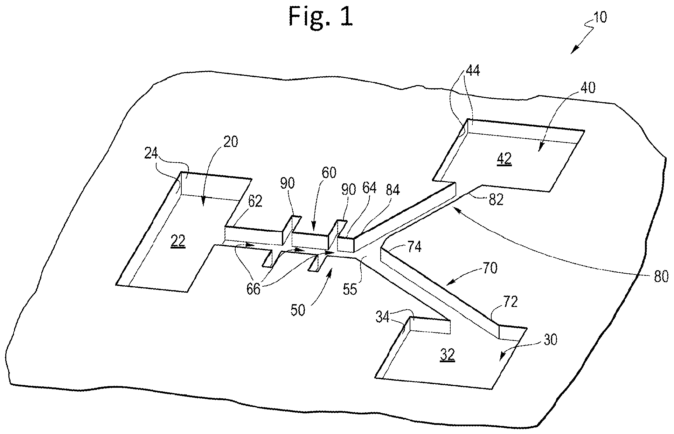

An exemplary embodiment of a biopsy tissue handling device 10 is shown in FIG. 1. The tissue handling device 10 includes a first well 20, a second well 30, and a third well 40. The first, second, and third wells 20, 30, and 40 each has, respectively, a bottom 22, 32, or 42, and a side wall 24, 34, or 44. In the embodiment in FIG. 1, a channel network 50 connects the first, second, and third wells 20, 30, and 40. The channel network 50 has a first channel 60, a second channel 70, and a third channel 80. Each of the first, second, and third channels 60, 70, and 80 has, respectively, a first end 62, 72, or 82, and a second end 64, 74, or 84. The first end 62 of the first channel 60 is connected to the first well 20. The first end 72 of the second channel 70 is connected to the second well 30. The first end 82 of the third channel 80 is connected to the third well 40. The second ends 64, 74, and 84, respectively, connect to form an intersection point 55. The first well 20, thus, is in fluid communication with the second and third wells 30 and 40 through the channel network 50 through channels 60, 70, and 80.

The first channel 60 in FIG. 1 includes a main branch 66 and optional transverse grooves 90 at spaced apart intervals. Although two grooves 90 are shown, the biopsy tissue handling device may include any number of grooves, depending on the particular application (e.g., any integer from 1 to 10).

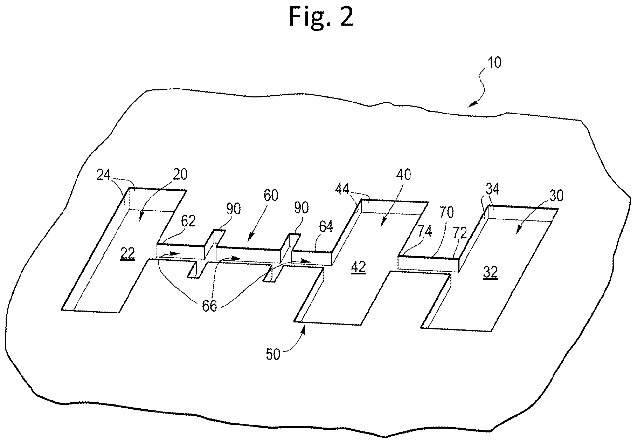

An alternate embodiment of a biopsy tissue handling device 10 is shown in FIG. 2. The tissue handling device 10 in FIG. 2 includes a first well 20, a second well 30, and a third well 40. The first, second, and third wells 20, 30, and 40 each has, respectively, a bottom 22, 32, or 42, and a side wall 24, 34, or 44. In the embodiment in FIG. 2, the third well 40 is positioned in the channel network 50 between the first and second wells 20 and 30. The channel network 50 includes a first channel 60 having first and second ends 62 and 64. The first end 62 is connected to the first well 20. The second end 64 is connected to the third well 40. The first channel 60 connects the first well 20 to the third well 40. The channel network 50 also includes second channel 70 having first and second ends 72 and 74. The first end 72 is connected to the second well 30. The second end 74 is connected to the third well 40. The second channel 70 connects the second and third wells 30 and 40. In the embodiment shown in FIG. 2, the third well 40 comprises a part of the fluid communication between the first and second wells 20 and 30.

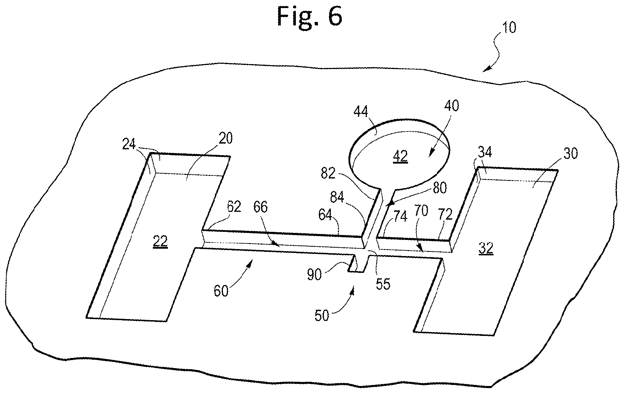

Another alternate embodiment of a biopsy tissue handling device 10 is shown in FIG. 6. The tissue handling device 10 in FIG. 6 includes a first well 20, a second well 30, and a third well 40. The first, second, and third wells 20, 30, and 40 each has, respectively, a bottom 22, 32, or 42, and a side wall 24, 34, or 44. In the embodiment in FIG. 6, a channel network 50 connects the first, second, and third wells 20, 30, and 40. The channel network 50 has a first channel 60, a second channel 70, and a third channel 80. Each of the first, second, and third channels 60, 70, and 80 has, respectively, a first end 62, 72, or 82, and a second end 64, 74, or 84. The first end 62 of the first channel 60 is connected to the first well 20. The first end 72 of the second channel 70 is connected to the second well 30. The first end 82 of the third channel 80 is connected to the third well 40. The second ends 64, 74, and 84, respectively, connect to form an intersection point 55. The channel network 50 in FIG. 6 includes a side groove 90 that is disposed at the intersection point 55 of the first, second, and third channels 60, 70, and 80.

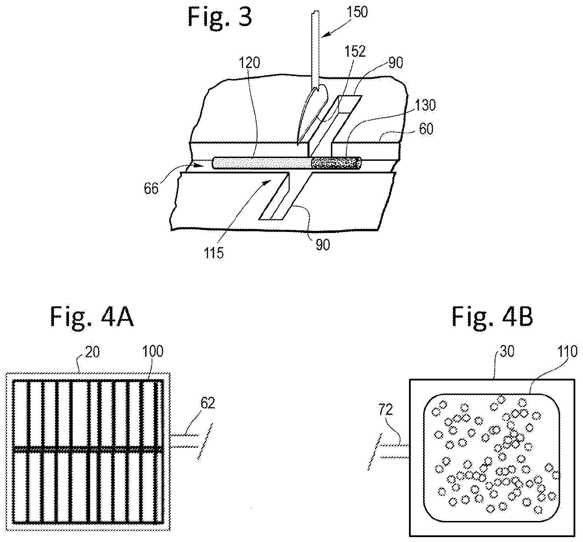

In FIG. 3 is shown a tissue specimen 115 made up of a tissue core 120 and a clot portion 130, with the tissue specimen 115 being positioned in the main branch 66 of a first channel 60. Also depicted is a cutting tool 150 having a cutting surface 152, the cutting surface 152 and the transverse groove 90 being sized such that the cutting surface 152 may be received within the transverse groove 90 when the cutting tool 150 is used to separate the tissue core 120 from the clot 130, such as by downward vertical movement of the cutting tool at the interface between the tissue core 120 and clot portion 130. The cutting surface 152 may be any implement having sufficient strength and rigidity to cut the tissue core 120 away from any clot material 130 (e.g., cutting blade, cutting wire, guillotine).

The first well 20 may include a grate or grill 100 (FIGS 4A and 5A) upon which a tissue specimen 115 may initially be positioned following transfer from a biopsy needle. A sterile solution 140 (e.g., saline) may be used in the first well 20 to bathe the tissue specimen 115 prior to further processing of the tissue specimen. FIG. 5A illustrates a tissue specimen 115 being transferred from the first well 20 to the first channel 60 of the channel network 50 while being bathed in a sterile solution 140. The grill 100 may aid in separating individual tissue specimens when multiple specimens are deposited within the first well 20. Alternatively, where the first well 20 has a depth greater than the first channel 60, the grill can serve to maintain a tissue specimen level with the bottom of the first channel. Alternatively, keeping the tissue specimen elevated allows blood and other fluids to seep through while keeping the core elevated.

The second well 30 may house a tissue receptacle or platform upon which a tissue core 120 may be positioned following processing in the channel network 50, where the core 120 is separated away from the clot 130. For example, the receptacle in the second well 30 may be a first sponge 110 (FIG. 4B and 5C) upon which a tissue core 120 may be positioned following processing in the channel network 50 to separate away the clot 130. Alternatively, the receptacle may be a piece of filter, other type of paper, a specimen cassette, a glass slide or other surface onto which the core may be positioned. In another variation, the second well 30 may also contain a grate or grill 100 onto which a piece of paper, a specimen cassette, a glass slide or other surface may be positioned. Generally, the receptacle may be any structure sized to fit the second well and suitable for transport of the core 120, including a pathology cassette 160, as discussed herein. A sterile solution 140 may be used in the second well 30 to bathe the tissue core 120 prior to transfer to a pathology lab.

Another alternate embodiment of a biopsy tissue handling device 10 is configured as a packaging tray to hold medical instruments used in conjunction with a system of wells and channels shown in the examples in FIGS. 1-6. A packaging tray according to the invention may have the form of a single integrally molded part containing the system of wells and channels, along with one or more holders configured to hold or otherwise immobilize a medical instrument, such as biopsy needle (e.g., 200), a syringe (e.g., 300), or tissue manipulation tools, for example, a tissue cutting tool (e.g., 150), or a tissue maneuvering/transferring tool (e.g., 170). In some embodiments, the biopsy kit of the invention includes the foregoing medical instruments and/or tissue manipulation tools. Alternatively, the packaging tray may be composed of more than one tray. For example, the system of wells and channels may be incorporated into a detachable biopsy tray that may be detached from a main tray. The detachable biopsy tray may be fitted into a depression in the main tray or may comprise a portion of the main tray that can be peeled or pulled away from the main tray.

Generally, the manner in which a medical instrument or tissue manipulation tool is held in the packaging tray is not critical. The medical instruments or tissue manipulation tools may be held in place with holders such as fasteners, ties, clips, recessed pockets, etc. Examples of packaging systems for medical devices may be found in the following US patent nos.: 4736850; 3851649 and 5031775. The configuration of the holders may be determined by the exact configuration of the specific medical instrument that it is designed to hold Recessed pockets may be molded into a packaging tray to releasably hold medical instruments placed into individual recessed pockets in the tray. Relative to the plane of the packaging tray, the recessed pockets may hold the medical instruments below the plane, in the plane, or above the plane. For example, elevated pockets may be molded onto a packaging tray such that the medical instruments may be held planar to the surface of the packaging tray or reside above the planar surface of the packaging tray. Elevated pockets may be designed to have a configuration suited to releasably hold a medical instrument that may be snapped into the elevated pocket. The pockets are generally in the shape of the particular instrument which they are designed to contain. It is not necessary that the pocket be shaped to mate exactly with the instruments which they are adapted to receive. The pocket may be of the same depth as most of the instruments that it is designed to receive or it may extend below the depth of the instrument so that a user may remove the instruments by reaching under the instruments held in the pocket. Alternatively, the pocket may have a shallower depth than the instruments such that part of the instruments projects beyond the pocket. Recessed pockets may be used in combination with other holders, e.g., ties, fasteners, etc.

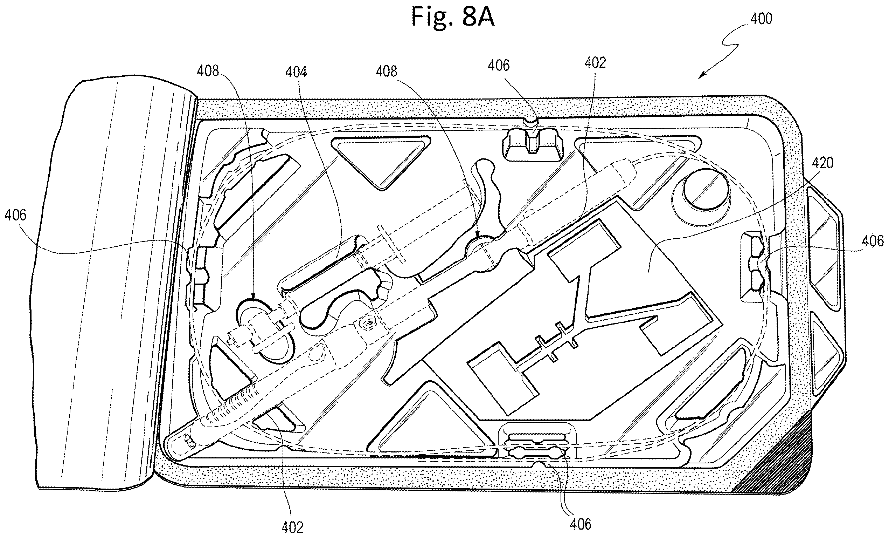

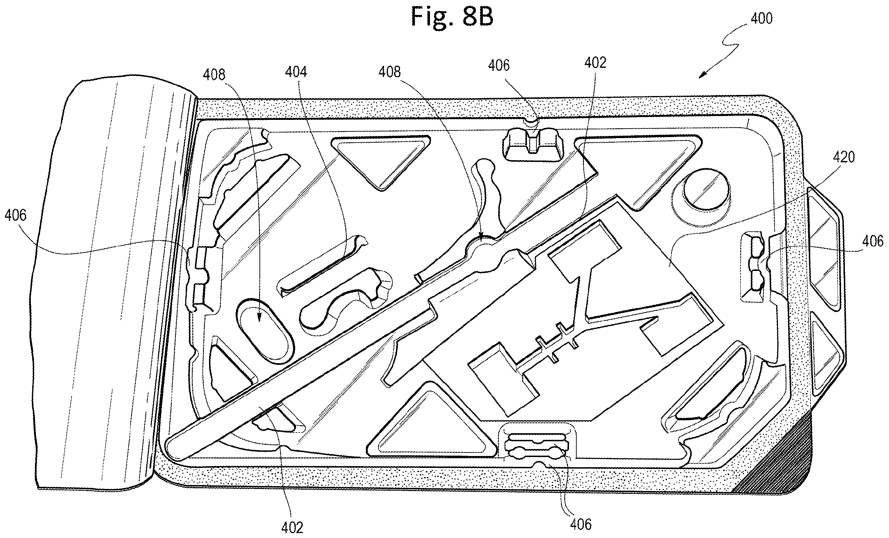

FIGS. 8A and 8B illustrate in detail an example of a packaging tray 400 with one or more pockets configured to receive a biopsy needle 200 and a syringe 300. An example biopsy needle 200 and syringe 300 are shown in FIGS. 7A and 7B and with dashed lines in a packaging tray 400 in FIG. 8A. In the embodiments of FIGS. 8A and 8B, the system of wells and channels is shown generally as 420 as part of a packaging tray 400. The system of wells and channels 420 is shown disposed adjacent to the pockets 402 and 404 for the needle and syringe. In FIGS. 8A and 8B, the sizes of the wells and channels 420 relative to the packaging tray 400 and medical instruments are not necessarily to scale and details of the wells and channels 420 are omitted for clarity. Recessed pocket(s) 402 are configured to receive and hold the needle 200 by the handle 202 and the sheath 206. A series of pockets/grooves 406 along the inside of the peripheral edge of the packaging tray 400 releasably hold the cannula 204 of the needle 200. Recessed pocket 404 represents a pocket configured for receiving and holding a syringe (e.g., 300) in place, for example around the barrel 302. Finger depressions 408 may be disposed along the pockets 402 and 404 and groove 406 for easy access to and for the removal of the instruments contained therein.

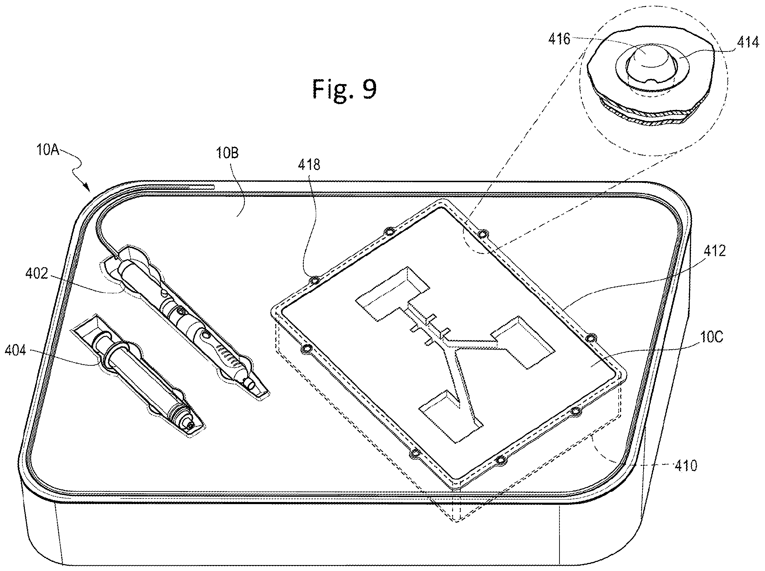

FIG. 9 shows an example of a packaging tray 10A having a main tray 10B and a detachable biopsy tray 10C, with a system of channels and wells. A depression 410 in the main tray 10B is configured to receive the detachable biopsy tray 10C that removably engages with the main tray 10B. As shown, each outer-edge 412 of the detachable biopsy tray 10C has one or more openings 414 that are designed to mate with a raised anchor 416 of the main tray 10B. In an embodiment, raised anchor 416 and opening 414 are each generally oval in shape, although opening 414 has a small tab 418 that improves the frictional lock between it and raised anchor 416. Alternatively, the respective positions of the anchors and openings could be reversed between the main tray 10B and detachable biopsy tray 10C. Alternatively, the anchors and opening maybe omitted and the detachable biopsy tray 10C could be sized to be press fit into the depression 410. As another alternative, the depression 410 may be replaced with a simple opening into which the detachable tray 10C may be fitted.

The biopsy tissue handling device may be made out of any suitable material that is compatible with pathology procedures including exposure to solvents and sterilization agents, e.g., plastics, polymers (e.g., polypropylene, PVC, PETG), glass, metal. Medical grade plastics may be used as described in McKeen, Handbook of Polymer Applications in Medicine and Medical Devices, 2014, Ch. 3, pp. 21-53. (http://dx.doi.org/10.1016/B978-0-323-22805-3.00003-7). The biopsy tissue handling device and kit may likewise be sterilized prior to use.

In operation, a tissue specimen 115 obtained from a biopsy needle may be transferred to the first well 20 of the biopsy tissue handling device 10. In the first well, the tissue specimen 115 may be deposited on an optional grate 100 bathed in sterile solution 140. The tissue specimen 115 may then be maneuvered into the first channel 60 of the channel network 50 for processing. In the first channel 60, the tissue specimen 115 may be positioned in the main branch 66 adjacent an optional transverse groove 90 such that the tissue core 120 and clot 130 are on opposite sides of the groove (FIG. 3). A cutting tool 150 may then be used to cut the tissue specimen 115 between the tissue core 120 and clot 130, the cutting surface 152 being accommodated within the groove 90 during the cutting movement. Alternatively, a cutting tool 150 may be utilized that is sized to fit within the main branch 66 of the first channel 60 without grooves 90, in which case, the grooves 90 may be omitted. Following separation of the tissue core 120 from the clot 130, the clot may be transferred to the third well 40 for later disposal and the tissue core 120 may be maneuvered into the second well 20 onto the optional first sponge 110, free of the clot material 130 (FIG. 5C). The first sponge 110 and tissue core 120 may then be removed from the second well 30 and transferred to a pathology cassette 160 (FIG. 5D) and covered with a second sponge 112 for transfer to a pathology lab. The pathology cassette 160 may also be fitted with a lid (not shown) to prevent accidental loss of the contents of the pathology cassette. The transfer/manipulation of tissue specimen 115/tissue core 120 to/within the biopsy handling device 10 may be carried out with the use of a tissue maneuvering device 170, which, for example may be an atraumatic tissue wand having a curved and/or flat tip to facilitate picking up a specimen/core.

An advantage of the invention over simple transfer of a tissue core from one container to another is that the biopsy tissue handling device allows processing of the delicate tissue specimen 115 with minimal lifting movements. The invention allows the tissue specimen 115 or tissue core 120 to be maneuvered through the channels of the channel network 50 such that the tissue core 120 may be deposited directly onto the sponge 110 located in the second well 30 without lifting the tissue core 120 and risking damage to the sample. Maintaining the integrity of the tissue core is expected to improve the quality and accuracy of the pathology assessment.

The dimensions of the wells, channels, and grooves of the biopsy tissue handling device may be sized as appropriate for the particular tissue processing application. Although the shapes of the wells (including bottoms and side walls), channel (including branches), and grooves are shown as generally square or rectangular, the shapes are not particularly important and may also be oval, round (as shown in well 40 in FIG. 6), rounded, curved triangular or other shape suitable for tissue processing operations. Likewise, the relative positions of the first, second, and third wells 20, 30, and 40, and angles of first, second, and third branches is not particularly important. Similarly, although the transverse grooves 90 are shown at right angles to the main branch 66 in FIGs. 1 and 2, the grooves may be oriented at any angle that allows clean separation of the tissue core 120 from the clot 130. The typical specimen sizes to be processed according to the invention range in length up to about 2 inches and may have a width/diameter from about 0.01 inches to about 0.05 inches. The width/diameter may be from about 0.015 to about 0.043 inches. Generally, the specimen width/diameter will correspond with the gauge of a biopsy needle, such as, for example, a biopsy needle from about 18 gauge to about 32 gauge (e.g., 18, 19, 20, 21, 22, 23, 24, 25 gauge). The dimensions of the wells and channels may be sized accordingly.

In one embodiment is provided a method of processing a tissue specimen comprising: (a) obtaining a tissue specimen 115 (e.g., a liver biopsy sample), the tissue specimen comprising a tissue core 120 and a clot 130; (b) transferring the tissue specimen 115 to a biopsy tissue handling device 10, the biopsy tissue handling device 10 comprising a channel network 50, a first well 20 and a second well 30, each well having a bottom, a side wall, and an upward facing opening, the first well 20 being in fluid communication with the second well 30 through the channel network 50, the tissue specimen 115 being transferred to the first well 20 of the biopsy handling device 10; (c) manipulating the tissue specimen to separate the tissue core 120 from the clot 130; and (d) transferring the separated tissue core through the channel network 50 to the second well 30.

In a sub-embodiment, the tissue specimen is separated into the tissue core and clot in the first channel.

In a sub-embodiment is provided a method where a tissue receptacle, such as a first sponge 110, is removably disposed in the second well 30 and the separated tissue core 120 is transferred through the channel network 50 onto the first sponge 110 in the second well 30. In other embodiments, other types of receptacles or platforms may be used other than the sponge to provide a surface onto which the core may be positioned, e.g., a piece of filter, other type of paper, a specimen cassette, a glass slide.

In a further sub-embodiment is provided a method where the biopsy tissue handling device 10 further comprises a third well 40, the third well having a bottom, a side wall, and an upward facing opening; and after separation of the tissue core from the clot, the third well receives the clot and the second well receives the tissue core.

In a further sub-embodiment is provided a method wherein the channel network comprises a main branch; and one or more transverse grooves, the one or more transverse grooves being in fluid communication with and disposed at an angle relative to the main branch. According to this sub-embodiment, the tissue core is separated from the clot at the one or more transverse grooves with a cutting tool having a cutting edge sized to fit in the one or more transverse grooves.

In a further sub-embodiment is provided a method wherein the channel network comprises: a first channel, the first channel having a first end and a second end, the first end being connected to the first well; a second channel, the second channel having a first end and a second end, the first end being connected to the second well; and a third channel, the third channel having a first end and a second end, the first end being connected to the third well; the second end of each of the first, second, and third channels being connected to form an intersection point. According to this sub-embodiment, after separation of the tissue core from the clot, the third well receives the clot through the third channel, and the second well receives the tissue core through the second channel. The embodiments of FIGS. 1 and 6 are suitable for use according to this sub-embodiment.

In a further sub-embodiment is provided a method wherein the channel network comprises: a first channel, the first channel having a first end and a second end, the first end being connected to the first well; and a second channel, the second channel having a first end and a second end, the first end being connected to the second well; the second end of each of the first and second channels being connected to the third well, such that the third well comprises an intersection point situated between the first and second wells, where the third well forms part of the fluid communication between the first and second wells. According to this sub-embodiment, after separation of the tissue core from the clot, the tissue core and clot may be transferred to the third well, and the tissue core then further transferred into the second channel and then into the second well. Thus, the second well receives the tissue core through the second channel, and the clot is received in the third well. The embodiment of FIG. 2 is suitable for use according to this sub-embodiment.

In a further sub-embodiment is provided a method wherein the first channel further comprises: a main branch; and one or more transverse grooves, the one or more transverse grooves being in fluid communication with and disposed at an angle relative to the main branch of the first channel. According to this sub-embodiment, the tissue core is separated from the clot at the one or more transverse grooves with a cutting tool having a cutting edge sized to fit in the one or more transverse grooves. The embodiments of FIGS. 1, 2, 3, and 6 are suitable for use according to this sub-embodiment.

In a further sub-embodiment is provided a method wherein the channel network comprises: a first channel, the first channel having a first end and a second end, the first end being connected to the first well; a second channel, the second channel having a first end and a second end, the first end being connected to the second well; a third channel, the third channel having a first end and a second end, the first end being connected to the third well; the second end of each of the first, second, and third channels being connected to form an intersection point; the channel network further comprising a side groove that is disposed at the intersection point of the first, second, and third channels. According to this sub-embodiment, the tissue core is separated from the clot at the side groove with the cutting tool being accommodated within the side groove and the intersection point, or optionally further within the first, second, or third channels, depending on the size of the cutting tool and the angle of the side groove relative to the individual channels. The embodiment of FIG. 6 is suitable for use according to this sub-embodiment.

In a further sub-embodiment is provided a method wherein the sponge, filter paper, glass slide, or other type of platform or receptacle and the separated tissue core are transferred from the second well to a pathology cassette.

The present invention is set out in the appended claims. The embodiments, aspects or examples according to the present description that do not fall within the scope of said claims are provided for illustrative purposes only and do not form part of the present invention.

## Claims

1. A biopsy kit comprising:
biopsy tissue handling device (10), comprising:
a first well (20), a second well (30), and a third well (40), each well having a bottom, a side wall, and an upward facing opening; and
a channel network (50);
the first well being in fluid communication with the second well and the third well through the channel network, wherein the channel network comprises a first channel (60), the first channel having a first end and a second end, the first end being connected to the first well, and **characterized in that** the first channel comprises one or more transverse grooves (90) in fluid communication with and disposed at an angle relative to the first channel, the one or more transverse grooves each being configured to accommodate a cutting tool therein.

2. The kit of claim 1, wherein the channel network further comprises:
a second channel, the second channel having a first end and a second end, the first end being connected to the second well; and
a third channel, the third channel having a first end and a second end, the first end being connected to the third well;
the second end of each of the first, second, and third channels connecting to form an intersection point.

3. The kit of claim 1, wherein the channel network further comprises:
a second channel, the second channel having a first end and a second end, the first end being connected to the second well; wherein the second end of each of the first and second channels is connected to the third well, such that the third well comprises a part of the fluid communication between the first and second wells.

4. The kit of any of claims 1-3, further comprising:
a grate (100), the grate being sized to fit within the first well.

5. The kit of any of claims 1-4, further comprising:
a tissue receptacle, the tissue receptacle being sized to fit in the second well.

6. The kit of any of claims 1-5, further comprising:
a cutting tool (150), the cutting tool having a cutting surface, the cutting surface being capable of cutting a tissue specimen positioned in the first channel at a location adjacent to one or more of the transverse grooves.

7. The kit of any of claims 1-6, further comprising:
a tissue maneuvering tool, the tissue maneuvering tool being capable of moving a tissue specimen, or part thereof, from the first well, through the channel network into the second well.

8. The kit of any of claims 1-7, wherein the biopsy tissue handling device is a biopsy tray.

9. The kit of any of claims 1-8, wherein the biopsy tissue handling device is a packaging tray, the packaging tray further comprising one or more holders configured to hold one or more of a biopsy needle, a syringe, a tissue manipulating tool, or a tissue cutting tool.

10. The kit of claim 9, wherein the packaging tray further comprises a detachable biopsy tray, the detachable biopsy tray comprising the first, second, and third wells, and the channel network.

11. The kit of claim 9 or 10, further comprising one or more of a biopsy needle, a syringe, a tissue manipulating tool, or a tissue cutting tool.

## Patentansprüche

1. Biopsie-Kit, umfassend:
Biopsiegewebehandhabungsvorrichtung (10), umfassend:
ein erstes Näpfchen (20), ein zweites Näpfchen (30) und ein drittes Näpfchen (40), wobei jedes Näpfchen einen Boden, eine Seitenwand und eine nach oben weisende Öffnung hat; und
ein Kanalnetz (50);
wobei das erste Näpfchen durch das Kanalnetz in Fluidverbindung mit dem zweiten Näpfchen und dem dritten Näpfchen steht, wobei das Kanalnetz einen ersten Kanal (60) umfasst, der ein erstes Ende und ein zweites Ende hat, wobei das erste Ende mit dem ersten Näpfchen verbunden ist, und
**dadurch gekennzeichnet, dass** der erste Kanal eine oder mehrere Quernuten (90) umfasst, die mit dem ersten Kanal in Fluidverbindung stehen und bezüglich dessen in einem Winkel angeordnet sind, wobei die eine oder mehreren Quernuten jeweils zur Aufnahme eines Schneidwerkzeugs darin ausgestaltet sind.

2. Kit nach Anspruch 1, wobei das Kanalnetz ferner Folgendes umfasst:
einen zweiten Kanal, wobei der zweite Kanal ein erstes Ende und ein zweites Ende hat, wobei das erste Ende mit dem zweiten Näpfchen verbunden ist; und
einen dritten Kanal, wobei der dritte Kanal ein erstes Ende und ein zweites Ende hat, wobei das erste Ende mit dem dritten Näpfchen verbunden ist;
wobei die jeweiligen zweiten Enden des ersten, zweiten und dritten Kanals miteinander verbunden sind, um einen Kreuzungspunkt zu bilden.

3. Kit nach Anspruch 1, wobei das Kanalnetz ferner Folgendes umfasst:
einen zweiten Kanal, wobei der zweite Kanal ein erstes Ende und ein zweites Ende hat, wobei das erste Ende mit dem zweiten Näpfchen verbunden ist, wobei das zweite Ende jedes des ersten und zweiten Kanals mit dem dritten Näpfchen verbunden ist, so dass das dritte Näpfchen einen Teil der Fluidverbindung zwischen dem ersten und dem zweiten Näpfchen umfasst.

4. Kit nach einem der Ansprüche 1 - 3, ferner umfassend:
ein Gitter (100), wobei das Gitter so bemessen ist, dass es in das erste Näpfchen passt.

5. Kit nach einem der Ansprüche 1 - 4, ferner umfassend:
eine Gewebeaufnahme, wobei die Gewebeaufnahme so bemessen ist, dass sie in das zweite Näpfchen passt.

6. Kit nach einem der Ansprüche 1 - 5, ferner umfassend:
ein Schneidwerkzeug (150), wobei das Schneidwerkzeug eine Schneidfläche hat, wobei die Schneidfläche in der Lage ist, eine im ersten Kanal an einer der einen oder den mehreren Quernuten benachbarten Stelle positionierte Gewebeprobe abzuschneiden.

7. Kit nach einem der Ansprüche 1 - 6, ferner umfassend:
ein Gewebemanövrierwerkzeug, wobei das Gewebemanövrierwerkzeug in der Lage ist, eine Gewebeprobe oder ein Teil davon aus dem ersten Näpfchen durch das Kanalnetz in das zweite Näpfchen zu bewegen.

8. Kit nach einem der Ansprüche 1 - 7, wobei die Biopsiegewebehandhabungsvorrichtung eine Biopsieschale ist.

9. Kit nach einem der Ansprüche 1 - 8, wobei die Biopsiegewebehandhabungsvorrichtung eine Verpackungsschale ist, wobei die Verpackungsschale ferner eine oder mehrere Aufnahmen umfasst, die dazu ausgestaltet sind, ein(e) oder mehrere einer Biopsienadel, einer Spritze, eines Gewebemanipulierwerkzeugs oder eines Gewebeschneidwerkzeugs aufzunehmen.

10. Kit nach Anspruch 9, wobei die Verpackungsschale ferner eine ablösbare Biopsieschale umfasst, wobei die ablösbare Biopsieschale das erste, zweite und dritte Näpfchen und das Kanalnetz umfasst.

11. Kit nach Anspruch 9 oder 10, ferner umfassend ein(e) oder mehrere einer Biopsienadel, einer Spritze, eines Gewebemanipulierwerkzeugs oder eines Gewebeschneidwerkzeugs.

## Revendications

1. Kit de biopsie, comprenant :
un dispositif de manipulation des tissus de biopsie (10), comprenant :
un premier puits (20), un deuxième puits (30), et un troisième puits (40), chaque puits ayant un fond, une paroi latérale, et une ouverture orientée vers le haut ; et
un réseau de canaux (50) ;
le premier puits étant en communication fluidique avec le deuxième puits et le troisième puits par le biais du réseau de canaux, le réseau de canaux comprenant un premier canal (60), le premier canal ayant une première extrémité et une deuxième extrémité, la première extrémité étant connectée au premier puits, et
**caractérisé en ce que**
le premier canal comprend une ou plusieurs rainures transversales (90) en communication fluidique avec le premier canal et disposées suivant un certain angle par rapport à celui-ci, la ou les rainures transversales étant chacune configurées pour recevoir un outil de coupe.

2. Kit selon la revendication 1, dans lequel le réseau de canaux comprend en outre :
un deuxième canal, le deuxième canal ayant une première extrémité et une deuxième extrémité, la première extrémité étant connectée au deuxième puits ; et
un troisième canal, le troisième canal ayant une première extrémité et une deuxième extrémité, la première extrémité étant connectée au troisième puits ;
les deuxièmes extrémités de chacun des premier, deuxième et troisième canaux étant connectées pour former un point d'intersection.

3. Kit selon la revendication 1, dans lequel le réseau de canaux comprend en outre :
un deuxième canal, le deuxième canal ayant une première extrémité et une deuxième extrémité, la première extrémité étant connectée au deuxième puits ; les deuxièmes extrémités de chacun des premier et deuxième canaux étant connectées au troisième puits de telle sorte que le troisième puits comprenne une partie de la communication fluidique entre les premier et deuxième puits.

4. Kit selon l'une quelconque des revendications 1 à 3, comprenant en outre :
une grille (100), la grille étant dimensionnée de manière à s'ajuster à l'intérieur du premier puits.

5. Kit selon l'une quelconque des revendications 1 à 4, comprenant en outre :
un réceptacle de tissus, le réceptacle de tissus étant dimensionné pour s'ajuster dans le deuxième puits.

6. Kit selon l'une quelconque des revendications 1 à 5, comprenant en outre :
un outil de coupe (150), l'outil de coupe ayant une surface de coupe, la surface de coupe étant capable de découper un spécimen de tissu positionné dans le premier canal en un emplacement adjacent à une ou plusieurs des rainures transversales.

7. Kit selon l'une quelconque des revendications 1 à 6, comprenant en outre :
un outil de manipulation de tissus, l'outil de manipulation de tissus étant capable de déplacer un spécimen de tissu ou une partie de celui-ci depuis le premier puits à travers le réseau de canaux jusque dans le deuxième puits.

8. Kit selon l'une quelconque des revendications 1 à 7, dans lequel le dispositif de manipulation des tissus de biopsie est un plateau de biopsie.

9. Kit selon l'une quelconque des revendications 1 à 8, dans lequel le dispositif de manipulation des tissus de biopsie est un plateau d'emballage, le plateau d'emballage comprenant en outre un ou plusieurs supports configurés pour retenir un ou plusieurs parmi une aiguille de biopsie, une seringue, un outil de manipulation des tissus ou un outil de coupe des tissus.

10. Kit selon la revendication 9, dans lequel le plateau d'emballage comprend en outre un plateau de biopsie détachable, le plateau de biopsie détachable comprenant le premier, le deuxième et le troisième puits, et le réseau de canaux.

11. Kit selon la revendication 9 ou 10, comprenant en outre un ou plusieurs parmi une aiguille de biopsie, une seringue, un outil de manipulation des tissus ou un outil de coupe des tissus.
